# FASCICULE DE BREVET EUROPEEN

(11) **EP 0 909 557 B1**
(45) Date de publication et mention de la délivrance du brevet: **03.12.2003**
(21) Numéro de dépôt: 98402271.5
(22) Date de dépôt: 15.09.1998
(51) Int. Cl.: A61K 7/48, A61K 7/06

(54) **Utilisation du miel en tant qu'agent diminuant l'adhésion des micro-organismes**
Verwendung von Honig zur Verminderung von Mikro-Organismenhaftvermögen
Use of honey as an agent for decreasing micro-organisms adhesion

(30) Priorité: 13.10.1997 FR 9712770
(43) Date de publication de la demande: 21.04.1999
(73) Titulaire: L'OREAL, 75008 Paris (FR)
(72) Inventeur: Breton, Lionel, 78000 Versailles (FR); Pineau, Nathalie, 19 rue du bas des Sables, 86000 Poitiers (FR)
(74) Mandataire: Tezier Herman, Béatrice

(56) Documents cités:
- DE-A- 2 058 226
- FR-A- 855 537
- US-A- 4 165 293
- "Bee products", rédacteurs: A. Mizrahi et al., édit.:Plenum Press, N.-Y,1996: S. GROBLER et al.:"The effect of honey on human tooth enamel and oral bacteria", XP002069864
- BASE DE DONN ES "CHEMICAL ABSTRACTS" (SERVEUR: STN), Abr. 110: 179 264, Colombus,Oh, USA; & JP 63 112 511 (A. KADOTA) 17 MAI 1988 XP002069869

## Description

L'invention se rapporte à l'utilisation du miel dans une composition cosmétique ou pour la préparation d'une composition pharmaceutique en tant qu'agent diminuant l'adhésion des micro-organismes, particulièrement des bactéries, sur la peau. En particulier les compositions de l'invention sont destinées à favoriser l'élimination des mauvaises odeurs corporelles ou à lutter contre toutes les infections cutanées mettant en jeu des bactéries, comme par exemple l'acné et/ou les pellicules.

Il est bien connu que la peau est couverte d'une flore responsable de toute une série de désagréments allant de la simple production d'odeur à des pathologies plus ou moins sévères comme par exemple l'acné et/ou les pellicules.

Les micro-organismes commensaux vivant sur ou dans la peau peuvent faire partie d'une microflore soit résidante (normale) soit transitoire. Les organismes résidants se développent normalement sur ou dans la peau. Leur présence est établie en profils de distribution bien définis. Les micro-organismes présents temporairement sont appelés transitoires. Habituellement, ces organismes ne se fixent pas fermement ; ils sont incapables de se multiplier et meurent normalement après quelques heures.

L'anatomie et la physiologie de la peau varient d'une partie à l'autre du corps et la microflore résidante reflète ces variations.
La plupart des bactéries de la peau sont présentes sur l'épiderme squameux superficiel, colonisant les cellules mortes ou étroitement associées aux glandes sébacées et sudoripares. Les excrétions de ces glandes fournissent de l'eau, des acides aminés, de l'urée, des électrolytes et des acides gras spécifiques servant d'éléments nutritifs principalement pour Staphylococcus epidermidis et des corynébactéries aérobies.
Les bactéries Gram-négatives sont généralement présentes dans les régions plus humides.
Les levures *Pityrosporum ovale et Pityrosporum orbiculare* sont normalement présentes sur l'épicrâne.
Certains mycètes dermatophytes peuvent coloniser la peau et provoquent des mycoses, comme par exemple le pied d'athlète et la teigne tonsurante.
Certains agents pathogènes présents sur ou dans la peau sont des résidants transitoires colonisant les zones autour des orifices. *Staphylococcus aureus* est le meilleur exemple. Il est présent dans les narines et la région périanale mais survit mal ailleurs. De la même manière, *Clostridium perfringens* colonise habituellement le périnée et les cuisses, particulièrement chez les patients souffrant du diabète.

Théoriquement, l'épiderme n'est pas un environnement favorable pour la colonisation par les micro-organismes. Plusieurs facteurs comme le dessèchement périodique de la peau, le pH légèrement acide de la peau, la concentration élevée en chlorure sodique de la sueur, certaines substances inhibitrices naturelles (bactéricides et/ou bactériostatiques) sont responsables de ce micro-environnement hostile.

L'absence d'humidité induit un état de dormance chez de nombreux résidants de la microflore. Cependant, sur certaines parties du corps (l'épicrâne, les oreilles, les régions axillaires, les régions génito-urinaire et anale, le périnée et les paumes), l'humidité est suffisamment élevée pour permettre l'existence d'une microflore résidante.
Le pH acide (4-6) de la peau, dû aux acides organiques produits par les staphylocoques et aux sécrétions des glandes sébacées et sudoripares, cécourage la colonisation par de nombreux micro-organismes.
La sueur contient du chlorure sodique en une concentration qui établit des conditions hyperosmotiques à la surface de la peau et pèse osmotiquement sur la plupart des micro-organismes.
Finalement, certaines substances inhibitrices naturelles aident à contrôler la colonisation, la croissance excessive et l'infection de la surface de la peau par les micro-organismes résidants. Par exemple, les glandes sudoripares excrètent du lysozyme qui lyse *Staphylococcus epidermidis* et d'autres bactéries Gram-positives. Certaines bactéries Gram-positives (*Propionibacterium acnes*) peuvent changer les lipides sécrétés par les glandes sébacées en acides gras insaturés, ccmme par exemple l'acide oléique, qui ont une forte activité antimicrobienne sur les bactéries Gram-négatives et les mycètes.

Cependant, dans certaines conditions ce système naturel de défense peut, tout en étant efficace, présenter des désagréments, voire être pris en défaut.

Par exemple certaines substances inhibitrices naturelles (bactéricides et/ou bactériostatiques) dues à la dégradation partielles des lipides complexes sécrétés par les glandes sudoripares sont volatils et peuvent être associés à une forte odeur que l'on a coutume de combattre. Certes de nombreux déodorants contiennent des substances antibactériennes agissant sélectivement sur les bactéries Gram-positives responsables de ces dégradations pour réduire la production d'acides gras insaturés aromatiques et l'odeur corporelle. Mais les déodorants peuvent modifier la microflore, principalement vers les bactéries Gram-négatives, et déclencher en conséquence des infections.

Il demeure donc intéressant de pouvoir disposer dans le cadre du traitement des odeurs corporelles de composés et/ou de compositions efficaces et ne présentant pas d'effets secondaires.

Un autre exemple est *Propionibacterium acnes*, la bactérie le plus frécuemment associée avec les glandes cutanées qui est un bâtonnet Gram-positif, anaérobie et lipophile. Cette bactérie est habituellement inoffensive. Toutefois, on l'a associée à une maladie cutanée, l'acné juvénile. L'acné apparaît ordinairement pendant l'adolescence lorsque le système endocrinien est très actif. L'activité hormonale stimule la surproduction de sébum, un fluide sécrété par les glandes sébacées. Un volume important de sébum s'accumule dans les glandes et fournit un micro-environnement idéal pour *Propionibacterium acnes.* Chez certains individus, cette accumulation déclenche une réponse inflammatoire provoquant une rougeur et un gonflement du canal glandulaire et produisant un comédon, un bouchon de sébum et de kératine dans le canal. Il en résulte des lésions inflammatoires (papules, pustules, nodules), communément appelées "points noirs". *Propionibacterium acnes* semble être l'organisme producteur de lipases qui dégradent les triglycérides du sébum en acide gras libres. Ces dérivés sont particulièrement irritants parce qu'ils peuvent pénétrer dans le derme et promouvoir une inflammation.

Les levures *Pityrosporum ovale* et *Pityrosporum orbiculare* sont communément associées à la formation des pellicules.

Les souches de *Staphylococcus aureus* sont connus comme super antigènes, ce qui favorise l'apparition de réactions d'irritation et de processus inflammatoires.

Bien évidemment, si nécessaire, on sait utiliser depuis longtemps des composés comme les antibiotiques ou certains dérivés naturels ou synthétique de la vitamine A pour palier aux déficiences de ce système naturel de défense.
Mais, là encore on sait que l'utilisation de tels composés présente des aspects négatifs non négligeables. L'utilisation abusive d'antibiotiques peut aboutir à l'émergence de micro-organismes résistants sur lesquels elles n'ont plus d'efficacité. Quant aux dérivés de la vitamine A, on sait qu'ils présentent généralement des effets secondaires importants ce qui rend leur utilisation délicate.
Ainsi, là encore on a besoin de composés et/ou de compositions efficaces et ne présentant pas d'effets secondaires.

Par ailleurs on sait qu'un des éléments clefs pour qu'un agent pathogène induise une maladie infectieuse est qu'il doit être capable d'adhérer à l'hôte qu'il va coloniser et envahir.

En effet après avoir été transmis à un hôte approprié, l'agent pathogène doit être capable de se fixer et de coloniser les cellules et les tissus de l'hôte. La colonisation dépend de la capacité qu'a le germe pathogène à concurrencer avec succès la microflore normale de l'hôte pour les éléments nutritifs essentiels. Des structures spécialisées, permettant d'entrer en concurrence pour des sites d'attachement en surface, sont également nécessaires à la colonisation.
Les organismes pathogènes, comme beaucoup de non pathogènes, se fixent de manière très spécifique à des tissus particuliers. Les facteurs d'adhérence sont un des éléments de cette spécificité. Ce sont des structures spécialisées présentes sur la surface de l'agent pathogène qui se fixent sur des récepteurs spécifiques des cellules hôtes.

On comprend donc qu'une des voies possibles pour traiter les désagréments et/ou les infections cutanées est de lutter contre l'adhésion des micro-organismes aux cellules de la peau et/ou des muqueuses.

De manière surprenante et inattendue, la demanderesse a découvert que le miel, et particulièrement le miel de fleurs d'acacias, présente la particularité de modifier l'attachement dés micro-organismes sur les cellules en particulier sur les cellules de la peau et/ou des muqueuses.

Il est connu d'utiliser le miel dans des compositions cosmétiques destinées à traiter le vieillissement. Ainsi, par exemple les documents JP-A-59-55809, GB-A-120673 et JP-A-6-38702 décrivent de telles compositions.

L'article de Bee Products (1996, 65-71) étudie l'effet de solutions de miel plus ou moins diluées sur la solidité de l'émail dentaire et sur la viabilité de différentes souches de la flore buccale. Les auteurs concluent qu'en dehors de S. anginosus et S. oralis, les souches orales de streptocoques, de même que C. albicans, sont relativement résistantes à l'activité antimicrobienne du miel.

DE 2058226 décrit une composition complexe comprenant un mélange de vaseline, acide salicylique, cires, goudron de pin, miel, camphre et térébenthine pour traiter l'eczéma, les durillons ou l'acné.

FR855537 se rapporte à une crème esthétique contenant de l'huile d'olive, de la cire blanche, du blanc de baleine et divers constituants dont du miel, pour hydrater la peau.

Mais, la capacité du miel à modifier l'attachement des micro-organismes sur les cellules en particulier sur les cellules de la peau et/ou des muqueuses n'a jamais été décrite à ce jour.

L'invention a donc pour objet l'utilisation, en tant que principe actif, dans une composition cosmétique ou pour la préparation d'une composition pharmaceutique d'une quantité efficace de miel, la composition étant destinée à modifier l'adhésion des micro-organismes sur la peau et/ou les muqueuses.

Par principe actif, on entend toute molécule ou composition susceptible de modifier ou de moduler le fonctionnement d'au moins un système biologique donné.

Par "modifier l'adhésion des micro-organismes" il faut entendre que la miel ou la composition le contenant peut être utilisé aussi bien à titre préventif, par sa capacité à prévenir, totalement ou partiellement, l'adhésion des micro-organismes, qu'à titre curatif par sa capacité à faciliter le détachement des micro-organismes.

Ainsi, l'invention a pour objet l'utilisation, en tant que principe actif, dans une composition cosmétique ou pour la préparation d'une composition pharmaceutique d'une quantité efficace de miel, la composition étant destinée à prévenir, totalement ou partiellement, l'adhésion des micro-organismes sur la peau et/ou les muqueuses.

De même l'invention a pour objet l'utilisation, en tant que principe actif, dans une composition cosmétique ou pour la préparation d'une composition pharmaceutique d'une quantité efficace de miel, la composition étant destinée à faciliter le détachement des micro-organismes de la peau et/ou les muqueuses.

Particulièrement le miel ou la composition le contenant est utilisé selon l'invention en application topique sur la peau et/ou les muqueuses.

On a vu précédemment que l'adhésion de micro-organismes à la peau et/ou aux muqueuses a des conséquences qui vont du simple désagrément (l'odeur) aux maladies plus ou moins graves.

Un des aspects de l'invention est donc de proposer l'utilisation du miel en tant qu'actif dans une composition cosmétique ou pour la préparation d'une composition pharmaceutique, la composition étant destinée aux soins d'hygiène corporelle, y compris les soins intimes.

Par soins d'hygiène corporelle on entend toutes substances ou préparations destinées à être mises en contact avec les diverses parties superficielles du corps humain et/ou avec les dents et/ou les muqueuses en vue de les nettoyer, de les protéger, de les maintenir en bon état, d'en modifier l'aspect, de les parfumer ou d'en corriger l'odeur.
Particulièrement, l'invention a pour objet l'utilisation du miel en tant qu'actif dans une composition cosmétique ou pour la préparation d'une composition pharmaceutique, la composition étant destinée à diminuer les mauvaises odeurs corporelles.

On a vu précédemment que la flore bactérienne de la surface de la peau est responsable d'un grand nombre de désordres.

Ainsi, l'invention a également pour objet l'utilisation du miel en tant qu'actif dans une composition cosmétique ou pour la préparation d'une composition pharmaceutique, la composition étant destinée à lutter contre les mycoses, l'acné, particulièrement l'acné juvénile et/ou les pellicules.

Particulièrement, l'invention a pour objet l'utilisation du miel en tant qu'actif dans une composition cosmétique ou pour la préparation d'une composition pharmaceutique, la composition étant destinée à lutter contre l'acné et/ou les pellicules.

On entend par miel le produit de la transformation par les abeilles du nectar et/ou du miellat des fleurs.

Bien que la teneur de ses différents constituants puisse varier selon sa provenance, le miel est généralement au moins un mélange de glucose, de lévulose, de maltose, de saccharose, ainsi que d'autres composants comme des protéines, des acides organiques, des lactoses, ds substances minérales, des oligo-éléments, des vitamines, de nombreux enzymes des substances aromatiques et bien évidemment de l'eau.
Le miel peut être un miel non fermenté ne contenant pas d'hydroxyacides ou un miel fermenté contenant des hydroxyacides.

Le miel peut être de toute provenance. Il peut s'agir notamment de miel de fleurs d'acacias, de tilleul, de lavande, de chataignier, de résineux, de fleurs d'oranger, de toutes fleurs de montagne, du "Gatinais".

Préférentiellement, on utilise selon l'invention du miel de fleurs d'acacias.

Par miel de fleurs d'acacias on entend tout miel obtenu à partir du nectar et/ou du miellat de fleurs de Robinier (*Robinia pseudacacia L.*).

La quantité de miel utilisable selon l'invention dépend bien évidemment de l'effet recherché et doit être en une quantité efficace pour empêcher partiellement, voire totalement, l'adhésion des micro-organismes ou pour faciliter le détachement des micro-organismes.

A titre d'exemple la quantité de miel utilisable selon l'invention peut aller par exemple de 0.1 % à 20 % et de préférence de 0,5 % à 10 % du poids total de la composition.

L'invention a également pour objet un procédé cosmétique pour traiter les désordres liés à l'adhésion de micro-organismes consistant à appliquer sur la peau une composition cosmétique comprenant du miel dans un milieu cosmétiquement acceptable.

Par milieu cosmétiquement acceptable, on entend compatible avec la peau, le cuir chevelu les muqueuses, les ongles, et les cheveux.

Elle peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin, comme produit de nettoyage, comme produit de maquillage ou encore comme simple produit déodorant.

Ainsi, l'invention a pour objet une composition cosmétique de soin, de nettoyage, de maquillage ou déodorante comprenant du miel.

Particulièrement l'invention a pour objet une composition cosmétique déodorante comprenant du miel.

Encore plus particulièrement la composition déodorante de l'invention est sous forme de stick.

De façon connue, la composition de l'invention peut contenir également les adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges, les filtres, les pigments, les agents chélateurs, les absorbeurs d'odeur et les matières colorantes. Les quantités de ces différents adjuvants sont celles classiquement utilisées dans les domaines considérés, et par exemple de 0,01% à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition de l'invention est une émulsion, la proportion de la phase grasse peut aller de 5 % à 80 % en poids, et de préférence de 5 % à 50 % du poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 % à 30 % en poids, et de préférence de 0,5 % à 20 % du poids total de la composition.

Comme huiles utilisables dans l'invention, on peut citer les huiles minérales, les huiles d'origine végétale (huile d'abricot, huile de tournesol), les huiles d'origine animale, les huiles de synthèse, les huiles siliconées (cyclométhicone) et les huiles fluorées (perfluoropolyéthers). On peut aussi utiliser comme matières grasses des alcools gras (alcool cétylique), des acides gras, des cires (cire d'abeilles).

Comme émulsionnants et coémulsionnants utilisables dans l'invention, on peut citer par exemple les esters d'acide gras et de polyéthylène glycol tels que le stéarate de PEG-40, le stéarate de PEG-100, les esters d'acide gras et de polyol tels que le stéarate de glycéryle et le tristéarate de sorbitane.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, on peut utiliser notamment les hydratants tels que les polyols (par exemple la glycérine), les vitamines (par exemple le D-panthénol), les agents anti-inflammatoires, les agents apaisants (allantoïne, eau de bleuet), les filtres UVA et UVB, les agents matifiants (par exemple les polydiméthylorganosiloxanes partiellement réticulés vendus sous le nom KSG® par Shin Etsu), et leurs mélanges.

On peut aussi ajouter des actifs antirides autres que le miel, et notamment des produits tenseurs tels que les protéines végétales et leurs hydrolysats, en particulier l'extrait de protéine de soja vendue sous le nom d'Eleseryl® par la société LSN ou le dérivé d'avoine vendu sous la dénomination Reductine® par la société Silab.

D'autres caractéristiques et avantages de l'invention ressortiront mieux des exemples qui suivent et des figures annexées, donnés à titre illustratif et non limitatif. Les proportions sont données en pourcentage pondéral.

### Exemple 1 : Comparaison de l'activité de différents miels dans le modèle de détachement cellulaire in vitro chez l'homme.

Ce test *in vitro* de screening d'un agent actif sur l'adhésion cellulaire est réalisé sur kératinocytes humains différenciés. Le principe du test repose sur le fait que l'inhibition de l'adhésion cellulaire induit la libération de kératinocytes humains différenciés.

Le pouvoir de détachement cellulaire du produit testé va être d'autant plus grand que le nombre de kératinocytes différenciés libérés sera important. Le protocole du test est le suivant : à partir de biopsies de peau humaine, après séparation de l'épiderme du derme, les kératinocytes sont dissociés par action enzymatique à la trypsine et mis en culture à la concentration de 2.10⁵ cellules/ml selon les techniques de culture cellulaires traditionnelles connues de l'homme du métier.
La croissance et la différenciation des kératinocytes est obtenue par culture durant 10 à 20 jours .

Après élimination du milieu de culture, l'activité du produit à tester est évaluée. Deux prélèvements de milieu de culture sont réalisés à T0 et T60, c'est à dire avant l'ajout du produit à tester (T0) et 60 minutes après cet ajout (T60). Les prélèvements ainsi effectués sont analysés au cytomètre de flux pour dénombrer la population de cornéocytes présents dans le milieu.
Le cytomètre de flux permet de distinguer les populations de cornéocytes et de kératinocytes par traitement à l'acridine orange spécifique de l'acide désoxyribonucléique (ADN) des cellules. Cette coloration est spécifique des kératinocytes puisque les cornéocytes ne possèdent pas de noyaux donc pas d'ADN.

Les résultats de ces études sont résumés dans le tableau suivant.

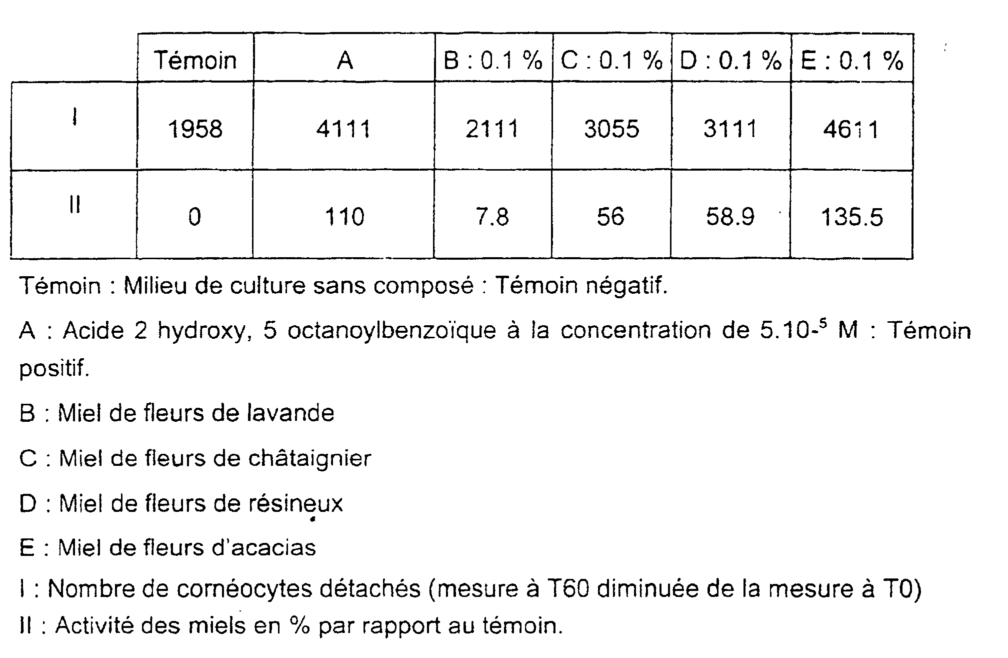

En conclusion: L'activité des miels et notamment du miel de fleurs d'acacias sur le détachement cellulaire varie en fonction de l'origine de celui-ci. Le miel de fleurs d'acacias présente la meilleure activité, au moins égal au témoin positif de référence.

Les résultats confirment l'activité inhibitrice du miel sur l'adhésion cellulaire. La provenance de celui-ci semble importante et le miel de fleurs d'acacias apparaît comme le meilleur inhibiteur de l'adhésion cellulaire

### Exemple 2 : Mesure de l'activité du miel sur l'adhésion de microorganismes sur la peau ou sur les muqueuses humaines.

Le miel de fleurs d'acacias, le plus actif dans le test précédent, a été retenu pour le test sur un modèle d'adhésion bactéries/peau, tel que décrit ci-après.

Ce modèle d'adhésion bactéries/peau *ex vivo* est basé sur l'utilisation de peau humaine intacte montée en sandwich entre une plaque 96 puits sans fond et un support Plexiglas. Les puits individualisés étanches permettent de tester dans un format standard l'effet du miel de fleurs d'acacias sur l'adhésion de *Staphylococcus epidermis* (bactéries résidentes) à la surface de la peau. Les bactéries sont radio marquées en phase de croissance par incorporation de 3H-thymidine. Cette flore bactérienne est ensuite déposée dans chaque puits. Après une heure d'incubation à 20°C, les puits sont lavés trois fois avec du tampon phosphate salin (PBS) ce qui permet d'éliminer les bactéries libres. Le miel de fleurs d'acacias est ensuite ajouté à 5 et 10% et incubé 1 heure à 20°C. A l'issue de cette incubation le contenu de chaque puits est récupéré et compté en scintillation liquide (bactéries désorbées). Les puits sont ensuite lavés avec une solution chaotropique qui permet d'éluer les bactéries adhérentes. Les solutions de lavages sont également compté en scintillation liquide (bactéries adhérentes). Les témoins positifs et négatifs (respectivement Fucogel® Acide (poly-[(α-1,3)-Fuc-(α-1,3)-Gal-(α-1,3)-galacturonique]) et Bioecolia® (Oligo-[α-1,4)-Gluc-(α-1,2)-Fruc-(α-1,6)-Gluc]) décrits dans la publication de Wolf F., et al. (Edition du 19^{ième} Congres IFSCC, Sydney, 1996), sont inclus à l'étude comme références.

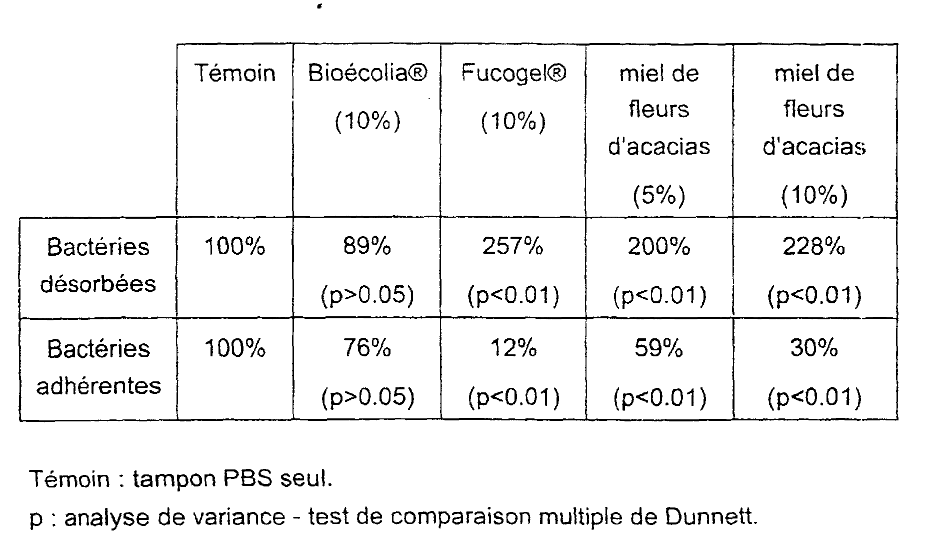

Le fucogel® (contrôle positif) stimule la désorption des bactéries et inhibe l'adhésion. Bioécolia® (contrôle négatif) ne modifie pas l'adhésion de façon significative. Le miel de fleurs d'acacias à 5 et 10% stimule la désorption des bactéries (228 et 200%) et inhibe l'adhésion (30 et 59%).

En conclusion, les résultats présentés ci-dessus confirment bien le rôle du miel de fleurs d'acacias dans la régulation de l'adhésion des bactéries sur la peau humaine. Cette activité est dose dépendante et identique à la celle de la référence positive.

### Exemple 3 : Exemples de compositions selon l'invention. Ces compositions sont obtenues par les techniques habituelles couramment utilisées en cosmétique ou en pharmacie.

### Composition 1 : Lait pour le visage

- Huile de vaseline 7 g
- miel de fleurs d'acacias 5 g
- Monostéarate de glycéryle, stéarate de polyéthylène glycol (100 OE) 3 g
- Polymère carboxyvinylique 0,4 g
- Alcool stéarylique 0,7 g
- Protéines de soja 3 g
- NaOH 0,4 g
- Conservateur qs
- Eau qsp 100 g

Cette composition se présente sous forme d'un lait pour le visage, ayant de bonnes propriétés cosmétiques, et étant doux et confortable à utiliser. Le pH de la composition est d'environ 5,5.

### Composition 2 : Lotion antiseptique

- miel de fleurs d'acacias 5,00 g
- Palmitate d'éthyl-2 hexyle 10,00 g
- Cyclopentadiméthylsiloxane 20,00 g
- Butylène glycol 5,00 g
- Conservateur qs
- Eau qsp 100 g

Cette lotion, qui ne contient pas de tensioactif, est particulièrement bien adaptée au lavage de la peau.

### Composition 3 : Lait

- Palmitate d'octyle 35,00 g
- Glycérine 2,00 g
- miel de fleurs d'acacias 5,00 g
- Polymère réticulé acrylates C10-C30/alkylacrylates 0,10 g
- Triéthanolamine 0,10 g
- Acides aminés de blé 1,00 g
- Conservateur qs
- Eau qsp 100 g

Le lait obtenu, qui ne contient pas de tensioactif, possède de bonnes propriétés cosmétiques.

### Composition 4 : Gel

- Glycérine 10,00 g
- miel de fleurs d'acacias 5,00 g
- Cocoamphodiacétate de disodium 1,00 g
- Conservateur qs
- Eau qsp 100 g

Le gel obtenu possède de bonnes propriétés cosmétiques.

### Composition 5 : Gel nettoyant à l'eau

- Butylène glycol 7,00 g
- Sarcosinate de lauroyl sodium 4,00 g
- miel de fleurs d'acacias 10,00 g
- Triéthanolamine 0,80 g
- Carbomer 0,50 g
- Conservateur qs
- Eau qsp 100 g

Le gel obtenu possède de bonnes propriétés cosmétiques.

### Composition 6 : Stick déodorant alcoolique

- Sodium stéarate 8,00 g
- Alcool éthylique 64,80 g
- Propylène glycol 10,00 g
- Myristate d'Isopropyle 5,00 g
- miel de fleurs d'acacias 12.20 g

### Composition 7 : Stick déodorant sans alcool

- Sodium stéarate 8,00 g
- Propylène glycol 10,00 g
- Coconut diéthanolamide 5,00 g
- PPG-3-Myristyl ether 65,00 g
- miel de fleurs d'acacias 12,00 g

## Revendications

1. Utilisation dans une composition cosmétique ou pour la préparation d'une composition pharmaceutique, en tant que principe actif, d'une quantité efficace de miel, la composition étant destinée à modifier l'adhésion des micro-organismes sur la peau et/ou des muqueuses.

2. Utilisation dans une composition cosmétique ou pour la préparation d'une composition pharmaceutique, en tant que principe actif, d'une quantité efficace de miel, la composition étant destinée à prévenir, totalement ou partiellement, l'adhésion des micro-organismes sur la peau et/ou des muqueuses.

3. Utilisation dans une composition cosmétique ou pour la préparation d'une composition pharmaceutique, en tant que principe actif, d'une quantité efficace de miel, la composition étant destinée à faciliter le détachement des micro-organismes de la peau et/ou des muqueuses.

4. Utilisation selon l'une quelconque des revendications précédentes, **caractérisée par le fait que** le miel ou la composition est utilisé en application topique sur la peau et/ou les muqueuses.

5. Utilisation dans une composition cosmétique ou pour la préparation d'une composition pharmaceutique, en tant que principe actif, d'une quantité efficace de miel selon l'une des revendications précédentes, la composition étant destinée aux soins d'hygiène corporelle.

6. Utilisation dans une composition cosmétique ou pour la préparation d'une composition pharmaceutique, en tant que principe actif, d'une quantité efficace de miel selon l'une des revendications précédentes, la composition étant destinée aux soins d'hygiène intime.

7. Utilisation dans une composition cosmétique ou pour la préparation d'une composition pharmaceutique, en tant que principe actif, d'une quantité efficace de miel, la composition étant destinée à diminuer les mauvaises odeurs.

8. Utilisation dans une composition cosmétique ou pour la préparation d'une composition pharmaceutique, en tant que principe actif, d'une quantité efficace de miel, la composition étant destinée à lutter contre les mycoses et/ou l'acné et/ou les pellicules.

9. Utilisation selon la revendication précédente, **caractérisée par le fait que** la composition est destinée à lutter contre l'acné juvénile.

10. Utilisation selon l'une quelconque des revendications précédentes, **caractérisée par le fait que** le miel est choisi parmi les miels d'acacias, de tilleul, de montagne, du "Gatinais", de fleurs d'oranger, de toutes fleurs.

11. Utilisation selon l'une quelconque des revendications précédentes, **caractérisée par le fait que** le miel est du miel de fleurs d'acacias.

12. Utilisation selon l'une quelconque des revendications précédentes, **caractérisée par le fait que** le miel est présent en une quantité allant de 0,1 % à 20 % du poids total de la composition.

13. Utilisation selon la revendication précédente, **caractérisée par le fait que** le miel est présent en une quantité allant de 0,5 % à 10 % du poids total de la composition.

14. Utilisation selon l'une quelconque des revendications précédentes, **caractérisée par le fait que** la composition se présente sous forme d'une lotion, d'un gel aqueux, d'un sérum, d'une émulsion ou d'une dispersion de vésicules lipidiques.

15. Utilisation selon l'une quelconque des revendications précédentes, **caractérisée par le fait que** la composition contient, en outre, au moins un adjuvant choisi parmi les gélifiants, les actifs, les conservateurs, les antioxydants, les solvants, les parfums, les charges, les filtres, les pigments, les agents chélateurs, les absorbeurs d'odeur, les matières colorantes, les agents matifiants et leurs mélanges.

16. Procédé de traitement cosmétique pour traiter les désordres liés à l'adhésion de micro-organismes consistant à appliquer sur la peau une composition cosmétique comprenant du miel dans un milieu cosmétiquement acceptable.

17. Composition cosmétique déodorante comprenant du miel.

18. Stick déodorant comprenant du miel.

## Claims

1. Use, in a cosmetic composition or for the preparation of a pharmaceutical composition, as active principle, of an effective amount of honey, the composition being intended to modify the adhesion of microorganisms to the skin and/or mucous membranes.

2. Use, in a cosmetic composition or for the preparation of a pharmaceutical composition, as active principle, of an effective amount of honey, the composition being intended to prevent, totally or partially, the adhesion of microorganisms to the skin and/or mucous membranes.

3. Use, in a cosmetic composition or for the preparation of a pharmaceutical composition, as active principle, of an effective amount of honey, the composition being intended to facilitate the detachment of microorganisms from the skin and/or mucous membranes.

4. Use according to any one of the preceding claims, **characterized in that** the honey or the composition is used as a topical application on the skin and/or mucous membranes.

5. Use, in a cosmetic composition or for the preparation of a pharmaceutical composition, as active principle, of an effective amount of honey according to one of the preceding claims, the composition being intended for body hygiene treatments.

6. Use, in a cosmetic composition or for the preparation of a pharmaceutical composition, as active principle, of an effective amount of honey according to one of the preceding claims, the composition being intended for intimate-hygiene treatments.

7. Use, in a cosmetic composition or for the preparation of a pharmaceutical composition, as active principle, of an effective amount of honey, the composition being intended to reduce unpleasant odours.

8. Use, in a cosmetic composition or for the preparation of a pharmaceutical composition, as active principle, of an effective amount of honey, the composition being intended to combat mycosis and/or acne and/or dandruff.

9. Use according to the preceding claim, **characterized in that** the composition is intended to combat juvenile acne.

10. Use according to any one of the preceding claims, **characterized in that** the honey is chosen from acacia honey, linden honey, mountain honey, honey from the Gâtinais region, orange-flower honey and honey from any flower.

11. Use according to any one of the preceding claims, **characterized in that** the honey is acacia-flower honey.

12. Use according to any one of the preceding claims, **characterized in that** the honey is present in an amount ranging from 0.1% to 20% of the total weight of the composition.

13. Use according to the preceding claim, **characterized in that** the honey is present in an amount ranging from 0.5% to 10% of the total weight of the composition.

14. Use according to any one of the preceding claims, **characterized in that** the composition is in the form of a lotion, an aqueous gel, a serum, an emulsion or a dispersion of lipid vesicles.

15. Use according to any one of the preceding claims, **characterized in that** the composition also contains at least one adjuvant chosen from gelling agents, active agents, preserving agents, antioxidants, solvents, fragrances, fillers, screening agents, pigments, chelating agents, odour absorbers, dyestuffs and matt-effect agents, and mixtures thereof.

16. Cosmetic treatment process for treating disorders associated with the adhesion of microorganisms, this process consisting in applying to the skin a cosmetic composition comprising honey in a cosmetically acceptable medium.

17. Deodorant cosmetic composition comprising honey.

18. Deodorant stick comprising honey.

## Patentansprüche

1. Verwendung von Honig in einer wirksamen Menge als Wirkstoff in einer kosmetischen Zusammensetzung oder für die Herstellung einer pharmazeutischen Zusammensetzung, wobei die Zusammensetzung dafür vorgesehen ist, die Adhäsion von Mikroorganismen auf der Haut und/oder den Schleimhäuten zu ändern.

2. Verwendung von Honig in einer wirksamen Menge als Wirkstoff in einer kosmetischen Zusammensetzung oder für die Herstellung einer pharmazeutischen Zusammensetzung, wobei die Zusammensetzung dafür vorgesehen ist, das Anheften von Mikroorganismen an die Haut und/ oder den Schleimhäuten vollständig oder teilweise zu verhindern.

3. Verwendung von Honig in einer wirksamen Menge als Wirkstoff in einer kosmetischen Zusammensetzung oder für die Herstellung einer pharmazeutischen Zusammensetzung, wobei die Zusammensetzung dafür vorgesehen ist, das Ablösen von Mikroorganismen von der Haut und/oder den Schleimhäuten zu erleichtern.

4. Verwendung nach einem der vorhergehenden Ansprüche, **dadurch gekennzeichnet, dass** der Honig oder die Zusammensetzung topisch auf der Haut und/ oder den Schleimhäuten angewendet wird.

5. Verwendung von Honig in einer wirksamen Menge nach einem der vorhergehenden Ansprüche als Wirkstoff in einer kosmetischen Zusammensetzung oder für die Herstellung einer pharmazeutischen Zusammensetzung, wobei die Zusammensetzung für Körperpflegeprodukte vorgesehen ist.

6. Verwendung von Honig in einer wirksamen Menge nach einem der vorhergehenden Ansprüche in einer kosmetischen Zusammensetzung oder für die Herstellung einer pharmazeutischen Zusammensetzung, wobei die Zusammensetzung für Intimpflegeprodukte vorgesehen ist.

7. Verwendung von Honig in einer wirksamen Menge als Wirkstoff in einer kosmetischen Zusammensetzung oder für die Herstellung einer pharmazeutischen Zusammensetzung, wobei die Zusammensetzung für die Beseitigung oder Verhinderung unangenehmer Gerüche vorgesehen ist.

8. Verwendung von Honig in einer wirksamen Menge als Wirkstoff in einer kosmetischen Zusammensetzung oder für die Herstellung einer pharmazeutischen Zusammensetzung, wobei die Zusammensetzung für die Bekämpfung von Mykosen und/oder Akne und/oder Schuppen vorgesehen ist.

9. Verwendung nach dem vorhergehenden Anspruch, **dadurch gekennzeichnet, dass** die Zusammensetzung für die Bekämpfung der Akne juvenilis vorgesehen ist.

10. Verwendung nach einem der vorhergehenden Ansprüche, **dadurch gekennzeichnet, dass** der Honig unter Akazienblütenhonig, Lindenhonig, Berghonig, "Gatinais"-Honig, Orangenblütenhonig und Blütenhonig ausgewählt wird.

11. Verwendung nach einem der vorhergehenden Ansprüche, **dadurch gekennzeichnet, dass** es sich bei dem Honig um Akazienblütenhonig handelt.

12. Verwendung nach einem der vorhergehenden Ansprüche, **dadurch gekennzeichnet, dass** der Honig in einer Menge enthalten ist, die im Bereich von 0,1 bis 20 % des Gesamtgewichts der Zusammensetzung liegt.

13. Verwendung nach dem vorhergehenden Anspruch, **dadurch gekennzeichnet, dass** der Honig in einer Menge enthalten ist, die im Bereich von 0,5 bis 10 % des Gesamtgewichts der Zusammensetzung liegt.

14. Verwendung nach einem der vorhergehenden Ansprüche, **dadurch gekennzeichnet, dass** die Zusammensetzung in Form einer Lotion, eines wäßrigen Gels, eines Serums, einer Emulsion oder einer Dispersion von Lipidvesikeln vorliegt.

15. Verwendung nach einem der vorhergehenden Ansprüche, **dadurch gekennzeichnet, dass** die Zusammensetzung außerdem mindestens einen Zusatzstoff enthält, der unter den Gelbildner, Wirkstoffen, Konservierungsmitteln, Antioxidantien, Lösemitteln, Parfüms, Füllstoffen, Pigmenten, Chelatbildnern, Geruchsabsorbern, Farbmitteln, Mattierungsmitteln und den Gemischen dieser Zusatzstoffe ausgewählt wird.

16. Verfahren zur kosmetischen Behandlung für die Behandlung von Störungen, die mit dem Anheften von Mikroorganismen verbunden sind, das darin besteht, auf die Haut eine kosmetische Zusammensetzung aufzutragen, die Honig in einem kosmetisch akzeptablen Medium enthält.

17. Kosmetische desodorisierende Zusammensetzung, die Honig enthält.

18. Deodorantstift, der Honig enthält.
